# EUROPEAN PATENT APPLICATION

(11) **EP 0 696 644 A1**
(43) Date of publication of application: **14.02.1996**
(21) Application number: 95112266.2
(22) Date of filing: 04.08.1995
(51) Int. Cl.: C12Q 1/56, B01L 3/14, G01N 33/49

(54) **Apparatus for inhibiting glycolysis in blood samples**

(30) Priority: 09.08.1994 US 287982
(71) Applicant: Becton Dickinson and Company, Franklin Lakes, New Jersey 07417-1880 (US)
(72) Inventor: Ikeda, Tatsuhiko, Kawasaki-shi, Kanagawa-ken (JP); Dastane, Ajit Nachiket, North Arlington, New Jersey 07031 (JP); Losada, Robert, New York, N.Y. 11105 (JP)
(74) Representative: von Kreisler, Alek, Dipl.-Chem.

(57) **Abstract**

A blood collection device comprising a formed additive particle formulation. The additive particle formulation is an improvement over available additive formulations that are powder blended in that the components of the additive formulation of the present invention are in each formed particle. The formed additive particle formulation comprises a fluoride salt and an oxalate salt to minimize glycolysis and coagulation of a blood specimen.

## Description

### BACKGROUND OF THE INVENTION

### 1. Field of the Invention

This invention relates to a blood collection device for use in chemistry studies. More particularly, the present invention pertains to a blood collection device comprising an additive formulation, which formulation has combined antiglycolysis and anticoagulation properties.

### 2. Description of the Related Art

In carrying out blood collection, centrifuging and measurement of the blood sugar value for a specimen, a series of steps are required. Since the steps required are time consuming due to the increased complexity of the collecting and testing work, efficiency tends to be poor.

When blood is allowed to remain in a tube after being collected, the blood sugar value of the specimen declines with the passage of time because of glycolysis, that is, consumption of the sugar component by the cells in the blood. Therefore, an additive or reagent for inhibiting glycolysis in blood that is collected and stored for a period of time prior to testing is needed.

A typical antiglycolytic agent, sodium fluoride has been used to reduce glycolysis in blood. Sodium fluoride is associated with antiglycolytic action as well as hemolytic toxicity and anticoagulant activity.

However, the anticoagulant activity of the sodium fluoride is not sufficient at the low additive levels required for antiglycolytic action and averting hemolysis. Therefore the blood sample treated with sodium fluoride only is not suitable for the analysis of the sugar content by methods affected by hemolysis in the plasma. Therefore, sodium fluoride used as an anticoagulant agent limits the subsequent method of blood sugar analysis. To remedy the shortcoming of sodium fluoride, another component such as potassium oxalate has been combined with the sodium fluoride so as to form an additive formulation for use in blood collection devices.

Freeze drying, vacuum drying, liquid filling and powder filling are the conventional methods for filling additive formulations into blood collection devices. However, these conventional methods have disadvantages. In the case of freeze-drying, additive formulations can be rehydrated again after drying, which is not desirable. With drying methods, the additive formulations tend to be localized within the tube. Also, vacuum drying process may adversely affect the dissolution characteristics of the additives.

Powder filling of formulations is currently used to produce additives that have more than two (2) components wherein the components are dry blended before filling into the tubes. However, it is very difficult to blend and fill because the component ratios vary due to the different specific gravity and particle size of each component. Powder mixing consists of sifting the components and then mixing in a high speed mixer. The result is a bulk powder mixture that is then dispensed into a tube. Each component separates from the other in the dry blended powder formulation by shock or vibration. As a result, the component ratio varies in each tube.

Liquid filling is not practical because of the low solubility of the antiglycolytic and anticoagulant components of the additives. Due to the low solubility large liquid fills are required which reduce the glucose value of the resulting plasma sample is by dilution. In addition, liquid filling is not practical because liquid additives are subject to permeation through plastic tubes which will lead to drying of the additive and poor dissolution characteristics.

A need has been identified for solid additive formulations with improved fill accuracy for blood collection devices. Blood collection tubes need to be designed in such a manner that additive fill formulations in tubes efficiently work in tests or analysis, and the formulations do not interfere with testing or analysis. Such tests include but are not limited to hematology, blood chemistry, blood typing, toxicology analysis and therapeutic drug monitoring.

### SUMMARY OF THE INVENTION

The present invention is a blood collection device with an additive formulation comprising a low solubility component, fluoride salt and a high solubility component, an anticoagulant, wherein the additive particle formulation has a mesh size of about 20 to about 120.

Preferably, the fluoride salt is sodium fluoride, lithium fluoride, potassium fluoride or ammonium fluoride and most preferably sodium fluoride.

Preferably, the anticoagulant is an oxalate salt such as potassium oxalate ammonium oxalate, sodium oxalate or lithium oxalate and most preferably potassium oxalate.

Preferably, the additive particle formulation comprises about 1 mg to about 10 mg of a fluoride salt and about 1 mg to about 10 mg of an oxalate salt per 1 ml of blood and most preferably about 2.5 mg of a fluoride salt and about 2 mg of an oxalate salt per 1 ml of blood.

The additive particle of the present invention is formulated by a method, comprising the following steps:
(a) mixing a fluoride salt and an anticoagulant;
(b) adding distilled water to the mixture;
(c) forming the mixture into particles;
(d) heat-drying the particles until the moisture content of the particles is less than about 0.5% excluding water or crystallization; and
(e) sieving the particles into a mesh size of about 20 to about 120.

The additive particles are then filled into a blood collection tube. Most preferably, the mesh size of the additive particles is about 42 to about 80.

Preferably, the forming step is spraying-drying, extruding, granulating and the like.

The form and grain size of the additive particles are uniform and therefore the disintegration and dissolution velocity also uniform. Therefore, the effects of the additive on the blood, namely its capacity to prevent glycolysis and coagulation are constant. It is believed that as the high solubility component of the particle dissolves, it disperses the lower solubility component into the blood specimen increasing its surface area available for dissolution.

With this invention, in contrast to the commercially available additives in tubes, the additive particle formulation of the present invention comprising a particle of a fluoride salt and an anticoagulant, having a mesh size of about 20 to 120, can be more easily filled into a blood collection tube. A blood collection tube with this particle formulation will provide the desired antiglycolysis and anticoagulation properties to the specimen collected.

Furthermore, the additive particle formulation of the present invention provides more consistent results as compared to the commercially available additive formulations. Commercially available additive formulations consist of blended powder formulations comprising sodium fluoride and potassium oxalate. In the blended powder mixtures, the density and particle size of each component varies, causing the component ratios to change during tube filling process.

Such commercially available products include VACUTAINER® Glass Tubes containing a powder fill of sodium fluoride (7.5 mg) and potassium oxalate (6 mg), 5:4 ratio, Order No. 367722 (Becton, Dickinson and Company, 1 Becton Drive, Franklin Lakes, New Jersey 07417-1880).

The additive particle formulations of the present invention provides better and more consistent contact with the blood specimen and therefore more rapid dissolution of the particle additive formulation into the blood specimen is facilitated and the initiation of glycolysis and coagulation of the blood specimen is prevented.

Most notably, the additive particle formulation of the present invention provides a more stable blood to additive concentration over the shelf life of the device so that the product performance over the shelf-life of the product remains more consistent.

Additionally, the additive particle formulation of the present invention have substantially improved flow characteristics as compared to a blended powder mixture of the same components.

Preparation and validation of a powder blend formulation is time consuming and does not provide consistent formulation. Furthermore, there is substantial material waste and homogeneity of the blend that is difficult to maintain. In addition, the dispensing process of milligram quantities of powder blend may result in individual tube aliquots which may differ significantly from the needed blend ratio.

Furthermore, there is a cost advantage with the device and the method for making the same according to the present invention. The increased precision in providing a particle additive formulation into the tube enables lower amounts of components to be used. Therefore, waste during manufacturing is minimized and cost reductions are realized.

The additive particle formulation improves collection and analysis of blood by a number a factors: (i) a direct sampling device nozzle or probe of an automatic analyzer is less likely to clog because the formation of fibrin in the plasma is reduced due to the improved anticoagulation of the sample; (ii) improved additive fill due to the consistency of the components in the fill; and (iii) improved dissolution rate of the low solubility component (fluoride salt), due to dispersion of the low solubility component within the high solubility component. As the high solubility component dissolves it disperses the low solubility component into the blood specimen increasing its surface area available for dissolution.

Thus the additive particle formulation of the present invention imparts to the collection devices and the samples contained therein, combined anticoagulation and antiglycolysis properties. In addition, the process of formulating the additive as a particle of components reduces the hygroscopic property of the oxalate salt in the particle which helps in the filling operation by improving fill accuracy and reducing fill variation of the additive into the blood collection device. As the oxalate salt absorbs moisture, it changes in weight and causes a reduction in the fill volume required. Furthermore, powder behave differently in the filling equipment. Therefore, there is a variation in the volume of powder dispensed and filling of the blood collection device.

Unlike the conventional fill processes, the present invention does not require preparing solutions, mixing powder or a drying procedure after filling additive. Thus preparation and control of the additive particle formulation is simple and work efficiency improves in production.

The present invention has improved additive fill due to the consistency of the components of the particle. The variation in fill of the fluoride salt and the anticoagulant is reduced since error is then limited to fill error and not the combination of fill error and segregation error. Segregation error is caused due to separation of chemical components on account of their density and particle size differences during process of filling. Additionally, the volumetric fill error itself is reduced by controlling the particle size range. Furthermore, reducing the overall variation in fill quantities of the subcomponents, improves specimen quality by maintaining optimal additive to blood ratios. Therefore, the performance of the additive particle is reliable and consistent.

### DESCRIPTION OF THE DRAWINGS

FIG. 1 is a perspective view of a typical blood collection tube with a stopper.

FIG. 2 is a longitudinal sectional view of the tube of FIG. 1 taken along line 2-2, comprising the particle additive formulation of the present invention.

### DETAILED DESCRIPTION

The present invention may be embodied in other specific forms and is not limited to any specific embodiments described in detail which are merely exemplary. Various other modifications will be apparent to and readily made by those skilled in the art without departing from the scope and spirit of the invention. The scope of the invention will be measured by the appended claims and their equivalents.

The additive particle formulation of the present invention preferably comprises a low solubility component and a high solubility component. Combining the additive components together increases the dispersion rate of the low solubility component due to the dispersion rate of the low solubility component within the high solubility component. Therefore, the surface area of the low solubility component is exposed and increased as the high solubility component dissolves in a blood specimen.

Most preferably, the low solubility component is a fluoride salt and the high solubility component is an anticoagulant.

Preferably, the fluoride salt is sodium fluoride, lithium fluoride, potassium fluoride or ammonium fluoride. Most preferably, the fluoride salt is sodium fluoride.

Preferably, the anticoagulant is an oxalate such as potassium oxalate, sodium oxalate, lithium oxalate or ammonium oxalate. Most preferably, the oxalate is potassium oxalate.

Preferably, the additive particle formulation, per 1 ml of blood, comprises:
(a) from about 1.0 to about 10.0 mgs of a fluoride salt; and
(b) from about 1.0 to about 10.0 mgs of an oxalate salt.

Most preferably, the additive particle formulation, per 1 ml of blood, comprises about 2.5 mg of a fluoride salt and about 2.0 mg of an oxalate salt.

Preferably the particle mesh size of the additive formulation is about 20 to about 120 and most preferably from about 42 to about 80.

The additive particle formulation is prepared by a method, comprising the following steps:
(a) mixing a fluoride salt and an oxalate salt;
(b) adding distilled water to the mixture;
(c) forming the mixture into particles;
(d) heat-drying the particles to a moisture content is less than about 0.5% to about excluding water of crystallization; and
(e) sieving the particles into a mesh size of about 20 to about 120.

The additive particles are then filled into a blood collection tube.

Preferably, the forming step is spraying-drying, extruding, granulating and the like.

Combining the additive components together increases the dissolution rate of the low solubility component (fluoride salt) due to the dispersion of the low solubility component within the high solubility component (oxalate salt). Therefore, the surface area of the low solubility component is exposed and increases as the high solubility component dissolves.

The advantages of a tube with the additive particle formulation of the present invention is more precise, uniform fill, stable test values minimizing glycolysis and coagulation of a blood specimen and good dissolution of the particle into a blood specimen. Furthermore, the exposure of the particle to a blood specimen is enhanced.

A blood collection device of the present invention can be either an evacuated blood collection device or a non-evacuated blood collection device. The blood collection device is desirably made of polyethylene terephtahlate or polypropylene.

Water is most preferably used as the solvent for mixing and forming the particle because water has minimal detrimental effects on product or environment.

A variety of other compounds can be formed into the particles. Such things include but are not limited to, polyvinylpyrrolidone and carboxymethyl cellulose.

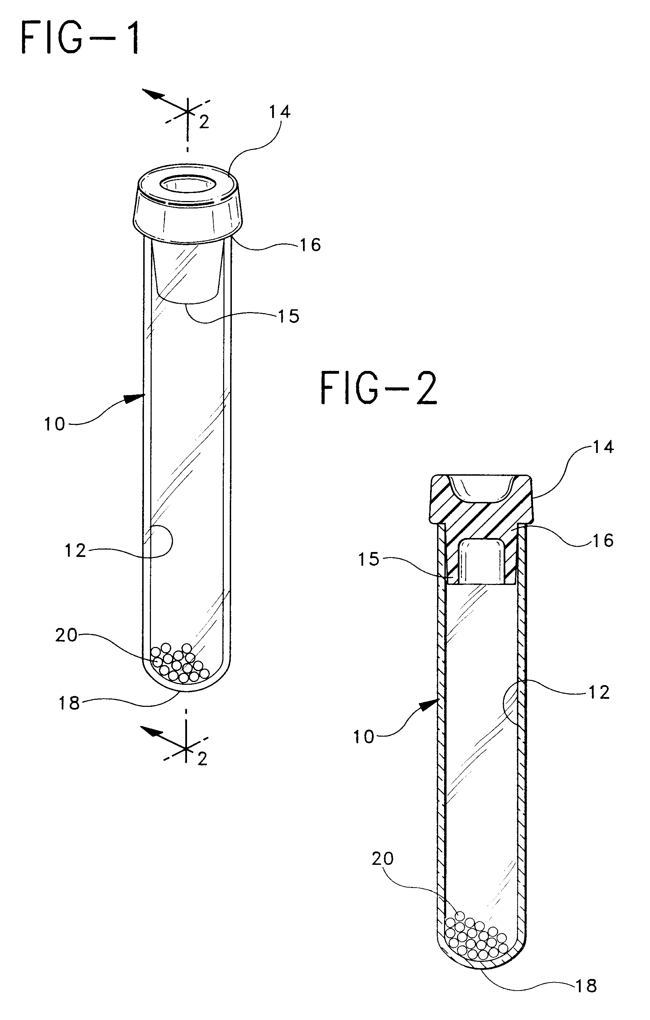

Referring to the drawings in which like parts throughout the several views thereof, FIG. 1 shows a typical blood collection tube **10**, having an open end **16**, a closed end **18**, and a stopper **14** that includes a lower annular portion or skirt **15** which extends into and processes against the inside wall **12** of the tube for maintaining stopper **14** in place.

FIG. 2 shows the particle of the formulation of the present invention in a typical blood collection tube. The particle additive formulation **20** is shown near the closed end of the tube.

A blood sample of interest can be transferred into tube **10** that comprises particle additive formulation **20**. The blood sample efficiently contacts particle additive formulation **20** so that particle additive formulation **20** rapidly dissolves into the blood sample and glycolysis and coagulation is substantially prevented for a period of time.

Various other modifications will be apparent to and may be readily made by those skilled in the art without departing from the scope and spirit of the invention.

The examples are not limited to any specific embodiment of the invention but are only exemplary.

### EXAMPLE 1

### PREPARATION OF ADDITIVE PARTICLE FORMULATION

An additive particle formulation was prepared by dissolving about 100g of sodium fluoride and about 80g of potassium oxalate in about 35 mls of distilled water. The aqueous formulation was then formed and dried into particles. The particles were sieved to a mesh size of about 42 to 80. In each tube, 9 mg of the particles of the formulation were placed.

The tubes were each evacuated to a 2ml draw and sealed with a closure and sterilized by gamma irradiation at 1.5 mega Rads.

## Claims

1. A blood collection device comprising a top end, a bottom end, a sidewall extending from said top end to said bottom end and including an exterior and interior surface and a granulated additive particle formulation comprising a low solubility component and a high solubility component wherein said particles have a mesh size from about 20 to about 120 and said high solubility component dissolves and dispenses said low solubility component into a blood specimen thereby exposing and increasing the surface area of the low solubility component for dissolution.

2. The device of Claim 1 wherein said low solubility component is a fluoride salt and said high solubility component is an oxalate.

3. The device of Claim 2 wherein said additive particle, per 1 ml of blood comprises:
(a) about 1.0 mg to about 10.0 mg of a fluoride salt; and
(b) about 1.0 mg to about 10.0 mg of an oxalate salt.

4. An additive particle formulation for use in blood collection tubes to minimize glycolysis and coagulation, comprising a fluoride salt and an oxalate salt, having a mesh size from about 20 to about 120.

5. The particle of Claim 4, per 1 ml of blood, comprising:
(a) about 1.0 mg to about 10.0 mgs of a fluoride salt; and
(b) about 1.0 mg to about 10.0 mgs of a oxalate salt.

6. A method for preparing an additive particle formulation comprising the steps of:
(a) preparing a mixture of a fluoride salt and an oxalate salt;
(b) dissolving said mixture in water;
(c) forming said mixture into particles;
(d) heat-drying said particles to a moisture content of less than about 0.5% excluding water of crystallization; and
(e) sieving said particles into a mesh size of about 20 to about 120 mesh size.

7. The method of Claim 6 wherein said forming step is spraying-drying, extruding or granulating.

8. The method of Claim 7 wherein said additive particle formulation, per 1 ml of blood, comprises:
(a) about 1.0 mg to about 10.0 mg of a sodium fluoride; and
(b) about 1.0 mg to about 10.0 mg of a potassium oxalate.

9. The method of Claim 8 wherein said particle, per 1 ml of blood comprises about 2.5 mg of a sodium fluoride and about 2.0 mg of a potassium oxalate.

10. The method of Claim 9 wherein the mesh size of said particle is from about 42 to about 80.
